# EUROPEAN PATENT SPECIFICATION

(11) **EP 2 072 505 B1**
(45) Date of publication and mention of the grant of the patent: **01.02.2012**
(21) Application number: 08170827.3
(22) Date of filing: 05.12.2008
(51) Int. Cl.: C07D 263/20

(54) **A process for the preparation of oxazolidinone derivatives**
Verfahren zur Herstellung von Oxazolidinonderivaten
Procédé de préparation de dérivés d'oxazolidinone

(30) Priority: 18.12.2007 IT MI20072359
(43) Date of publication of application: 24.06.2009
(73) Proprietor: Dipharma Francis S.r.l., 20021 Baranzate (MI) (IT)
(72) Inventor: Colombo, Lino, 27100 PAVIA (IT); Allegrini, Pietro, 20097 S. DONATO MILANESE (MI) (IT); Brusasca, Marco, 15048 VALENZA PO (AL) (IT); D'Arienzo, Giuseppe, 82100 BENEVENTO (IT); Razzetti, Gabriele, 20099 SESTO SAN GIOVANNI (MI) (IT)
(74) Representative: Minoja, Fabrizio

(56) References cited:
- WO-A-95/07271

## Description

### FIELD OF THE INVENTION

The present invention relates to a process for the preparation of antibacterial oxazolidinone derivatives, particularly [(S)-N-[[3-(3-fluoro-4-morpholinyl)phenyl]-2-oxo-5-oxazolidinyl]methyl]acetamide], and to the intermediates useful for the synthesis thereof.

### TECHNOLOGICAL BACKGROUND

Antibacterial oxazolidinone derivatives are known from WO 95/07271, which specifically describes the synthesis of linezolid, namely [(S)-N-[[3-(3-fluoro-4-morpholinyl)phenyl]-2-oxo-5-oxazolidinyl]methyl]acetamide], according to the following scheme:

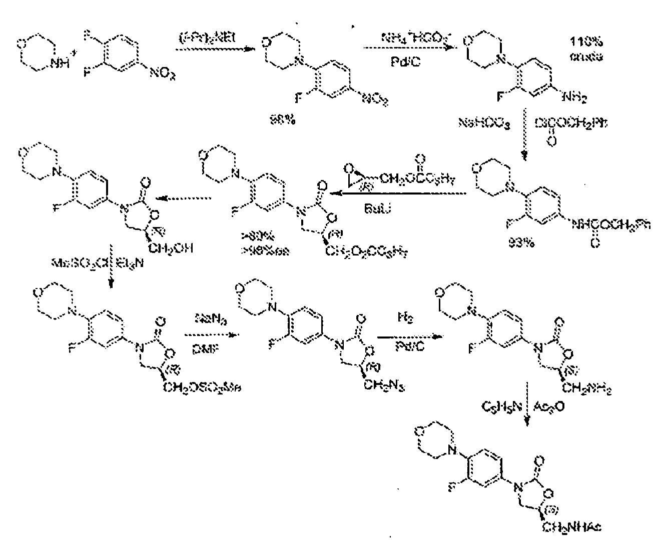

Other synthetic routes for the preparation of linezolid are reported for example in US 6107519 and in Tetrahedron Letters, Vol 37, N° 44, pages 7937-7940, wherein the chiral compound shown below is used instead of glycidyl butyrate as a synthon containing the molecule stereocenter.

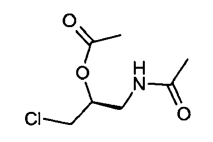

It should be appreciated that all of the known approaches to the preparation of linezolid make use of chiral synthons for the construction of the stereocenter. These are small molecules characterized by a high cost, therefore they are not suitable for the production of the compound on an industrial scale.

There is therefore the need for an alternative synthesis which provides oxazolidinone derivatives, linezolid included, from inexpensive starting materials, and which does not require a chiral synthon for the construction of the molecule, so that it can be used for the industrial preparation of such derivatives.

### BRIEF DISCLOSURE OF THE ANALYTICAL METHODS

The ¹H-NMR spectra were recorded with the Varian Mercury 300/Bruker 400 spectrometer, using DMSO-d6 or deuterated chloroform as the solvent.

The particles size was determined with the known laser light scattering technique using a Malvern Mastersizer MS 1 instrumentation under the following operative conditions:
- 300RF mm lens with of 2.4 mm laser beam length;
- sample of 500 mg dispersed in 10 ml of hexane (ACS reagent) with 1% SPAN 85^{®}, without presonication, and 2500 rpm stirring rate.

### DETAILED DISCLOSURE OF THE INVENTION

An object of the invention is a process for the preparation of a compound of formula (**I**), or a salt thereof, both as the single (S) or (R) enantiomer, and as a mixture thereof, 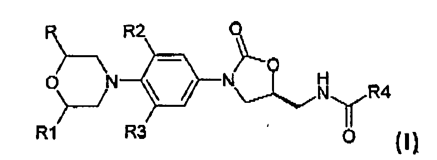 wherein R and R₁, which can be the same or different, are hydrogen, CH₃, CN, COOH or COOC₁-C₄ alkyl;
R₂ and R₃, which can be the same or different, are hydrogen, F or Cl; and
R₄ is C₁-C₄ alkyl;
which process comprises:
- the reduction of a compound of formula (**III**), or a salt thereof, as the single (S) or (R) enantiomer, or as a mixture thereof, 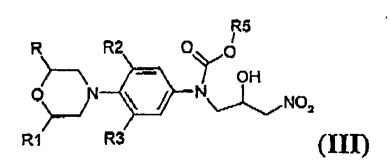 wherein R₅ is optionally substituted C₁-C₄ alkyl, aryl-C₁-C₄ alkyl or aryl; and R-R₄ are as defined above; to obtain a compound of formula (**IV**), or a salt thereof, as the single (S) or (R) enantiomer, or as a mixture thereof, 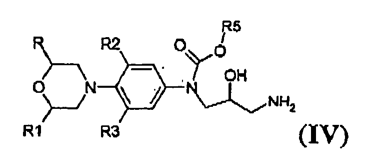 wherein R to R₃ and R₅ are as defined above;
- the acylation thereof to obtain a compound of formula (**II**), as the single (S) or (R) enantiomers, or as a mixture thereof, or a salt thereof, 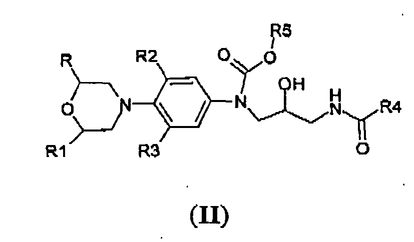 wherein R to R₅ are as defined above and, if the case; its conversion to a salt thereof, or *vice versa;* and
   a) the activation of the hydroxyl group in a compound of formula (**II**), as the single (S) or (R) enantiomer, or as a mixture thereof, or a salt thereof, by means of a derivatization reagent to form a leaving group, and
      subsequent cyclization reaction in the presence of a catalyst or an azide compound; or
   b) the cyclization reaction of a compound of formula (**II**), or a salt thereof, as the single (S) or (R) enantiomer, or as a mixture thereof, in presence of a strong base; and, if desired, the resolution of a mixture of enantiomers of formula (**I**) to the single (S) or (R) enantiomer; and/or, if desired, the conversion of a compound of formula (**I**) to a salt thereof, or *vice versa,* characterized in that
   c) a compound of formula (**III**), or a salt thereof, as a mixture of the two enantiomers, is obtained starting from an achiral compound of formula (V), or a salt thereof, 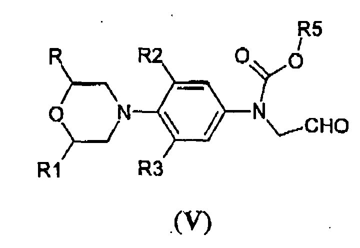 wherein R₁ to R₃ and R₅ are as defined above;
      by reaction with nitromethane in the presence of a base and, optionally, in the presence of a solvent; or
   d) a compound of formula (**III**), or a salt thereof, as a single (S) or (R) enantiomer is obtained starting from an achiral compound of formula (**V**), or a salt thereof,
   by reaction with nitromethane in the presence of an optically pure catalyst and, optionally, in the presence of a solvent; and
   if desired, the resolution of a mixture of the enantiomers of formula (**I**) to obtain the single enantiomer (S) or (R); and/or, if desired, the conversion of a compound of formula (**I**) to a salt thereof, or *vice versa.*

A C₁-C₄ alkyl group or residue, which can be straight or branched, is preferably methyl, ethyl, propyl, isopropyl, n-butyl or isobutyl or t-butyl; in particular methyl or ethyl or t-butyl.

An aryl-C₁-C₄ alkyl group is, for example, phenyl-C₁-C₄ alkyl or naphthyl-C₁-C₄ alkyl; in particular phenyl-C₁-C₂ alkyl, preferably benzyl.

An aryl group is, for example, phenyl or naphthyl; in particular phenyl.

An alkyl group or residue, when substituted, is substituted with 1 to 5 substituents, preferably 1 or 2, independently selected from hydroxy, C₁-C₆ dialkyl-amino, nitro, cyano and halogen.

An aryl group, when substituted, is substituted with 1 to 5 substituents, preferably 1 or 2, independently selected from hydroxy, C₁-C₆ alkyl, C₁-C₆ dialkyl-amino, nitro, cyano and halogen.

Preferably, in a compound of formula (**I**), in particular as the single (S) enantiomer, R, R₁ and R₃ are hydrogen; R₂ is fluorine; and R₄ is methyl. Preferably, in a compound of formula (**II**), R₅ is benzyl, t-butyl, isopropyl, isobutyl, methyl or ethyl; more preferably t-butyl when using alternative a) of the process of the invention, or ethyl when using alternative b).

A derivatizing agent can be for example a sulfonyl chloride of formula R₅SO₂Cl, wherein R₅ is as defined above, e.g. methanesulfonyl chloride or toluenesulfonyl chloride; or an alkyl or aryl phosphine, e.g. triphenylphosphine, and an azadicarboxylate, e.g. diethylazadicarboxylate; or a carbonyl diimidazole. Preferably methanesulfonyl chloride; or triphenylphosphine and diethyl-azadicarboxylate (Mitsunobu system).

A leaving group thus obtained is an easily displaceable hydroxyl-derived group, preferably a mesylate group.

A catalyst can be a Lewis acid, for example BF_{3,} AlCl₃, ZnCl₂, trifluoromethanesulfonic acid, trifluoroacetic acid, acetic acid, trimethylsilyl triflate, tert-butyldimethylsilyl triflate. Alternatively, an azide compound can be used, e.g. sodium azide but in stoichiometric amount

A strong base can be a strong organic or inorganic base, in particular a alkali C₁-C₆ alkoxide, e.g. sodium or potassium methoxide, ethoxide or t-butoxide; a tertiary amine, e.g. 1,4-diazabicyclo[2.2.2]octane or 1,8-diazabicyclo[5.4.0]-undec-7-ene, or diisopropylethylamine; a carbanion, e.g. butyl lithium or hexyl lithium; an azanion, e.g. lithium diisopropylamide or lithium tetramethylpiperidide; sodium hydride; sodium or potassium hexamethyldisilazide. Preferably sodium hydride or potassium t-butoxide.

According to alternative a) of the process of the invention, when the compound of formula (**II**) is the (S) enantiomer, the resulting product of formula (**I**) has (R) configuration. When the compound of formula (**II**) is the (R) enantiomer, the resulting product of formula (**I**) has (S) configuration. When the compound of formula (**II**) is a mixture of the two enantiomers, the resulting product of formula (**II**) is a mixture of the two enantiomers.

According to alternative b), when the compound of formula (**II**) is the (S) enantiomer, the resulting product of formula (**I**) has (S) configuration. When the compound of formula (**II**) is the (R) enantiomer, the resulting product of formula (**I**) has (R) configuration. When the compound of formula (**II**) is a mixture of the two enantiomers, the resulting product of formula (**I**) is a mixture of the two enantiomers.

According to alternative a), the compound of formula (**II**) preferably has (R) configuration, whereas according to alternative b), the compound of formula (**II**) preferably has (S) configuration.

The cyclization reaction of a compound of formula (**II**), according to alternatives a) and b), can be carried out in the presence of an organic solvent, selected from e.g. a dipolar aprotic solvent, typically dimethylformamide, dimethylacetamide, acetonitrile, dimethylsulfoxide; an ether, typically diethyl ether, methyl-tert-butyl ether, tetrahydrofuran or dioxane; a chlorinated solvent, typically dichloromethane, chloroform or chlorobenzene; an apolar solvent, typically an aliphatic hydrocarbon, e.g. hexane or cyclohexane, or an aromatic hydrocarbon, typically benzene or toluene; a carboxylic acid, typically acetic acid or trifluoroacetic acid, or an alkanol, typically methanol, ethanol, isopropanol or n-butanol. The reaction is preferably carried out in tetrahydrofuran or dioxane, in particular when the base is sodium hydride; or in an alcohol, preferably ethanol, when the base is an alkoxide.

The reaction can be carried out at a temperature approx. ranging from -15°C to the reflux temperature of the solvent, preferably at room temperature.

The separation of a mixture of enantiomers of the compound of formula (**I**) into the single (S) and (R) enantiomers can be carried out according to known methods, for example by crystallization of a diastereomeric salt of addition with a strong acid such as camphorsulfonic acid, mandelic acid, tartaric acid, dibenzoyltartaric acid.

A compound of formula (**I**), or a salt thereof, in particular linezolid, obtained according to the process of the invention, has purity equal to or higher than 95%; preferably equal to or higher than 99%; more preferably equal to or higher than 99.7%.

A salt of a compound of formula (**I**) is for example a pharmaceutically acceptable salt, in particular a pharmaceutically acceptable acid addition salt.

A compound of formula (**I**) can be converted to a salt, and *vice versa*, according to known methods.

The purity of the resulting product can be increased by crystallization of a salt of addition with a mineral acid, such as hydrochloric acid or sulfuric acid. If desired, said salts can be cleaved by treatment with a basic agent, for example sodium hydroxide, to obtain the product as the free base, in particular linezolid.

The crystallization of a linezolid salt and its subsequent cleavage is a useful method for the purification of linezolid. This method is novel and is a further object of the present invention.

The resulting Linezolid, or a salt thereof, has particle mean size D₅₀ ranging from about 10 to 250 micrometres; said size can be further reduced by a fine grinding process according to known techniques.

The compounds of formula (**II**), both as the single (S) or (R) enantiomers, and as mixtures thereof, as well as the salts thereof, are novel, and are a further object of the invention.

Preferred examples of compounds of formula (**II**) are:
- ethyl N-(3-acetamido-2-hydroxy-propyl)-N-(3-fluoro-4-morpholinophenyl) carbamate;
- t-butyl N-(3-acetamido-2-hydroxy-propyl)-N-(3-fluoro-4-morpholinophenyl) carbamate; and
- isobutyl N-(3-acetamido-2-hydroxy-propyl)-N-(3-fluoro-4-morpholinophenyl) carbamate.

A compound of formula (**II**), or a salt thereof, both as the single (S) or (R) enantiomer, and as a mixture thereof, can be obtained by a process comprising the reduction respectively of the (R) or (S) enantiomer, or of the mixture thereof, of a compound of formula (**III**), or a salt thereof, 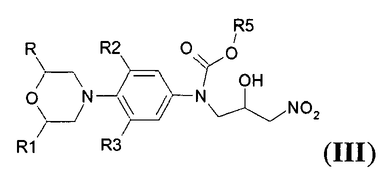 wherein R-R₅ are as defined above; to obtain a compound of formula (**IV**), or a salt thereof, both as the single (S) or (R) enantiomer, and as a mixture thereof, 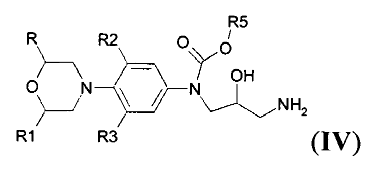 wherein R-R₅ are as defined above; the acylation thereof; and, if the case, the conversion to a salt thereof, or *vice versa*.

The reduction reaction of a compound of formula (**III**), or a salt thereof, can be carried out for example by catalytic hydrogenation in the presence of a homogeneous or heterogeneous metal catalyst, e.g. based on Pd, Pt, Ni, Rh or Ru; preferably based on Pd;

When the metal catalyst is heterogeneous, it is preferably deposited on an inert support, such as charcoal, barium hydroxide, alumina, calcium carbonate; preferably charcoal.

The concentration of the metal on the support can approximately range from 1 to 30%, preferably from about 5 to 10%.

The hydrogen pressure used can approximately range from 1 atm to 10 atm; the reaction is preferably carried out at atmospheric pressure.

The molar amount of catalyst used, based on the compound of formula (**II**), or a salt thereof, approximately ranges from 0.1 to 10%, preferably approximately from 0.5 to 5%.

The reaction can be carried out in the presence of an organic solvent, selected e.g. from a dipolar aprotic solvent, typically dimethylformamide, dimethylacetamide, acetonitrile, dimethylsulfoxide; an ether, typically tetrahydrofuran, dioxane or methyl-tert-butyl ether; an apolar solvent, e.g. an aliphatic hydrocarbon, typically hexane or cyclohexane, or an aromatic hydrocarbon, typically benzene or toluene; an alkanol, e.g. a C₁-C₄ alkanol, typically methanol, ethanol, isopropanol or butanol; an ester, typically ethyl acetate, isopropyl acetate, butyl acetate; a ketone, typically acetone, methyl ethyl ketone, methyl isobutyl ketone; a carboxylic acid, typically acetic acid or propionic acid; or mixtures of two or more, preferably two or three, of said solvents. Alternatively, the reaction can be carried out in water or an aqueous solution of a mineral acid, e.g. hydrochloric acid or sulfuric acid, or mixtures thereof with one, two or three of the organic solvents mentioned above. The reaction is preferably carried out in a C₁-C₄ alkanol, in particular methanol.

Alternatively, the reduction reaction of a compound of formula (**III**), or a salt thereof, can be carried out by hydrogen transfer reaction, using a homogeneous or heterogeneous metal catalyst, for example as reported above, substantially in the same molar amounts; and a hydrogen donor, optionally in the presence of a solvent.

A hydrogen donor can be selected e.g. from the group comprising cyclohexene, cyclohexadiene, methylcyclohexene, limonene, dipentene, mentene, hydrazine, phosphinic acid or derivatives thereof, e.g. sodium hypophosphite, indoline, ascorbic acid, formic acid or the sodium or ammonium salts thereof, and secondary alcohols, e.g. isopropanol. Cyclohexene or ammonium formate are preferred.

The molar ratio of the hydrogen donor to the compound of formula (**III**), or a salt thereof, can approximately range from 3 to 50, preferably from 3 to 10.

A solvent can be an organic solvent, selected from e.g. the solvents mentioned above for the reduction of the same compound of formula (**III)**, or mixtures of two or three of said solvents or with water.

The acylation reaction of a compound of formula (**IV**), or a salt thereof, to give a compound of formula (**II**) can be carried out according to known methods, using e.g. a C₁-C₄ acyl chloride or an anhydride as the acylating agent.

The stoichiometric ratio of the acylating agent to the compound of formula (**IV**) can approximately range from 0.8 to 1.2.

If desired, the reduction and the acylation can be carried out at the same time or in succession without isolating the compound of formula (**IV**).

The compounds of formula (**III**) and (**IV**), both as the single (R) or (S) enantiomers, and as a mixture thereof, and the salts thereof, are novel. Examples of compounds of formula (**III**) are:
- ethyl N-(3-fluoro-4-morpholinophenyl)-N-(2-hydroxy-3-nitropropyl) carbamate;
- isobutyl N-(3-fluoro-4-morpholinophenyl)-N-(2-hydroxy-3-nitropropyl) carbamate; and
- t-butyl N-(3-fluoro-4-morpholinophenyl)-N-(2-hydroxy-3-nitropropyl) carbamate;
both as the single (R) or (S) enantiomers, and as mixtures thereof.

Examples of compounds of formula (**IV**) are:
- ethyl N-(3-amino-2-hydroxypropyl)-N-(3-fluoro-4-morpholinophenyl)-carbamate;
- isobutyl N-(3-amino-2-hydroxypropyl)-N-(3-fluoro-4-morpholinophenyl)-carbamate; and
- t-butyl N-(3-amino-2-hydroxypropyl)-N-(3-fluoro-4-morpholinophenyl)-carbamate;
both as the single (R) or (S) enantiomers, and as mixtures thereof.

A compound of formula (**III**), or a salt thereof, can be obtained starting from an achiral compound of formula (**V**), or a salt thereof, 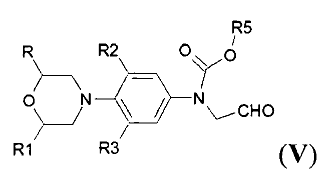 wherein R, R₁, R₂, R₃ and R₅ have the meanings reported above. In particular:
- a compound of formula (**III**), or a salt thereof, as a mixture of the two enantiomers can be obtained starting from an achiral compound of formula (**V**), or a salt thereof, by reaction with nitromethane in the presence of a base and, optionally, in the presence of a solvent; or
- a compound of formula (**III**), or a salt thereof, as the single (R) or (S) enantiomer, can be obtained starting from an achiral compound of formula (**V**), or a salt thereof, by reaction with nitromethane in the presence of an optically pure chiral catalyst and, optionally, in the presence of a solvent.

A base can be an organic base, for example tetrabutylammonium fluoride or diazabicycloundecene.

An optically pure chiral catalyst can be for example a complex of Cu(II) with a bisoxazolidine, in particular (3aR,3a'R,8aS,8a'S)-2,2'-(propane-2,2-diyl)bis8(8,8a-dihydro-3aH-indeno[1,2-d]oxazole) (1R,2S)-Inda-Box, prepared according to the procedure reported in Tetrahedron Letters, 45 (2004), pages 145-148.

A solvent can be, for example, an organic solvent selected from those reported above; preferably a C₁-C₄ alkanol; more preferably ethanol.

The molar ratio of nitromethane to a compound of formula (V), or a salt thereof, can range from 1 to 20, preferably about 10.

The molar ratio of the base to a compound of formula (V), or a salt thereof, can range from 0.01 to 1, preferably about 0.5.

The molar ratio of the chiral catalyst and to a compound of formula (V), or a salt thereof, can approximately range from 0.005 to 0.2; preferably approximately from 0.01 to 0.1.

A compound of formula (**II**) or (**III**), or a salt thereof, as the single (R) or (S) enantiomer, obtained according to the processes herein reported, has enantiomeric purity equal to or higher than 90%; said purity can be further increased by techniques known to those skilled in the art. Linezolid obtained starting from a compound of formula (**II**), or a salt thereof, with said enantiomeric purity characteristics, can have similar enantiomeric purity characteristics, namely equal to or higher than 90%, particularly equal to or higher than 99.5%.

The compounds of formula (**V**), and the salts thereof, are novel.

Examples of compounds of formula (**V**) are:
- ethyl 3-fluoro-4-morpholinophenyl-(2-oxoethyl)-carbamate;
- isobutyl 3-fluoro-4-morpholinophenyl-(2-oxoethyl)-carbamate; and
- t-butyl 3-fluoro-4-morpholinophenyl-(2-oxoethyl)-carbamate.

A compound of formula (**V**), or a salt thereof, can be prepared for example starting from a compound of formula (**VI**), or a salt thereof, by the process reported in Scheme A, 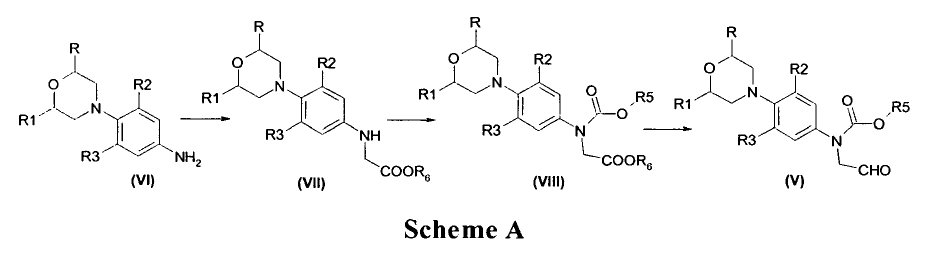 wherein R₆ is a straight or branched C₁-C₄ alkyl group, preferably methyl, and R, R₁, R₂, R₃ and R₅ have the meanings reported above, comprising:
- the reaction of a compound of formula (**VI**) with a bromoacetate of formula BrCH₂COOR₆, optionally in the presence of a base, e.g. sodium or potassium carbonate, to give a compound of formula (**VII**), or a salt thereof;
- the reaction of a compound of formula (**VII**), or a salt thereof, with a chloroformate of formula R₅OCOCl, optionally in the presence of a base, e.g. a tertiary amine, to give a compound of formula (**VIII**), or a salt thereof; and
- the reduction of a compound of formula (**VIII**), or a salt thereof, with diisobutylaluminium hydride to give a compound of formula (**V**), or a salt thereof.

The reactions represented in Scheme A are known and can be carried out according to known methods.

Alternatively, a compound of formula (**V**), or a salt thereof, can be prepared starting from a compound of formula (**VI**), or a salt thereof, by a process, reported in Scheme B, 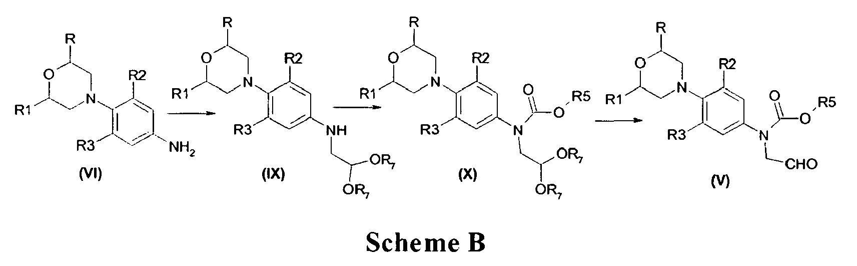 wherein each R₇ is independently C₁-C₆ alkyl, which can be straight or branched, preferably a C₁-C₄ alkyl group; more preferably methyl or ethyl, or the two R₇ groups taken together form a 5- or 6- membered ring, and R, R₁, R₂, R₃ and R₅ have the meanings reported above, which process comprises:
- the reaction with a dialkoxyacetaldehyde of formula CHOCH(OR₇)₂, wherein R₇ is as defined above, in the presence of hydrogen and a catalyst; or with chloroacetaldehyde dialkylacetal of formula ClCH₂CH(OR₇)₂, wherein R₇ is as defined above, and a base, e.g. diisopropylethylamine, to give a compound of formula (**IX**), or a salt thereof;
- the reaction of a compound of formula (**IX**), or a salt thereof, with a chloroformate R₅OCOCl or a carbonate R₅OCOOR₅, optionally in the presence of a base, e.g. a tertiary amine, to give a compound of formula (**X**), or a salt thereof; and
- the hydrolysis of the acetal function in a compound of formula (**X**), or a salt thereof, with a mineral or organic acid, e.g. hydrochloric acid or trifluoroacetic acid, to give a compound of formula (**V**), or a salt thereof.

A dialkoxyacetaldehyde of formula CHOCH(OR₇)₂ is preferably dimethoxyacetaldehyde.

A chloroacetaldehyde dialkylacetal of formula ClCH₂CH(OR₇)₂, can be, for example, chloroacetaldehyde dimethylacetal or chloroacetaldehyde diethylacetal; preferably chloroacetaldehyde diethylacetal.

The reactions represented in Scheme B are known, and can be carried out according to known methods.

A salt of a compound of formula (**II**), (**III**), (**IV**), (**V**), (**VI**), (**VII**), (**VIII**), (**IX**) or (**X**) is for example a pharmaceutically acceptable salt, in particular a pharmaceutically acceptable acid addition salt.

The compounds of formula (**VII**), (**VIII**), (**IX**), (**X**) and the salts thereof are novel.

Examples of compounds of formula (**VII**), (**VIII**), (**IX**) and (**X**) are:
- methyl2-(3-fluoro-4-morpholinophenylamino)acetate;
- methyl 2-((3-fluoro-4-morpholinophenyl)(isobutoxycarbonyl) amino) acetate;
- methyl 2-((3-fluoro-4-morpholinophenyl)(terbutoxycarbonyl)-amino) acetate;
- methyl 2-((3-fluoro-4-morpholinophenyl)(ethoxycarbonyl) amino) acetate;
- N-(2,2-diethoxyethyl)-3-fluoro-4-morpholinoaniline;
- N-(2,2-dimethoxyethyl)-3-fluoro-4-morpholinoaniline;
- ethyl 2,2-dimethoxyethyl(3-fluoro-4-morpholino-phenyl) carbamate;
- isobutyl 2,2-dimethoxyethyl(3-fluoro-4-morpholino-phenyl) carbamate;
- tert-butyl 1 2,2-diniethoxyethyl(3-fluoro-4-niorpholino-phenyl) carbamate;
- ethyl 2,2-diethoxyethyl(3-fluoro-4-morpholino-phenyl) carbamate;
- isobutyl 2,2-diethoxyethyl(3-fluoro-4-morpholino-phenyl) carbamate; and
- tert-butyl 2,2-diethoxyethyl(3-fluoro-4-morpholino-phenyl) carbamate.

A compound of formula (**VI**) is known and can be prepared according to the procedure reported in WO 95/07271.

The following examples illustrate the invention.

### Example 1: Synthesis of methyl 2-(3-fluoro-4-morpholino-phenylamino)acetate (VII; R=R₁=R₃=H; R₂=F; R₆=CH₃)

A solution of a 3-fluoro-4-morpholinoaniline (200 mg, 1.019 mmol), of formula (**VI**), in CH₃CN (5 ml) is added with K₂CO₃ (282 mg, 2.03 mmol), methyl bromoacetate (100 µl, 1.22 mmol) and a catalytic amount of tetrabutylammonium iodide. The mixture is microwave heated in a closed vessel, at about 150°C for approximately 20 minutes. Afterwards, acetonitrile is evaporated off, the solid is taken up with water and the mixture is extracted 3 times with ethyl acetate. The combined organic phases are dried over Na₂SO₄ and evaporated under reduced pressure. The product is then purified by flash chromatography (hexane:acetate 6:4), to obtain a white solid.

¹H NMR (400 MHz, *CDCl*₃) ppm 6.87 (t, *J* = 8.9 Hz, 1H), 6.42-6.33 (m, 2H), 4.21 (bs, 1H), 3.90-3.85 (m, 6H), 3.81 (s, 3H), 3.02-2.96 (m, 4H).

### Example 2: Synthesis of methyl 2-((3-fluoro-4-morpholino-phenyl) (isobutoxycarbonyl)amino)acetate (VIII; R=R₁=R₃=H; R₂=F; R₅=i.But; R₆=CH₃)

A solution of methyl 2-(3-fluoro-4-morpholinophenylamino)acetate (100 mg, 0.372 mmol), of formula (**VII**), in 1,2-dichloroethane (3.72 ml) is added with a catalytic amount of dimethylaminopyridine, triethylamine (50 µl, 0.372 mmol) and isobutyl chloroformate (100 µl, 0.744 mmol). The mixture is microwave heated in a closed vessel, at about 150°C for approximately 30 minutes, then a further 2 equivalents of isobutyl chloroformate are added, heating at the same temperature and under the same conditions for about 30 minutes. Afterwards, the mixture is diluted with CH₂Cl₂ and the organic phase is washed with a NaHCO₃ aqueous solution. The organic phases are dried over Na₂SO₄ and evaporated under reduced pressure. The product is purified by flash chromatography (hexane:acetate 7:3) to obtain a white solid in 80% yield.

¹H NMR (400 MHz, *CDCl*₃) ppm 7.16-7.00 (bm, 2H), 6.95-6.85 (m, 1H), 4.31 (s, 2H), 3.99-3.85 (m, 6H), 3.78 (s, 3H), 3.13-3.06 (m, 4H), 2.02-1.78 (bm, 1H), 1.01-0.78 (bm, 6H).

According to a similar procedure, the following compounds are obtained:
- methyl 2-((3-fluoro-4-morpholinophenyl)(ethoxycarbonyl)amino) acetate; and
- methyl2-((3-fluoro-4-morpholinophenyl)(terbutoxycarbonyl)-amino) acetate.

### Example 3: Synthesis of methyl 2-((3-fluoro-4-morpholinophenyl) (t-butoxycarbonyl)amino) acetate (VIII; R=R₁=R₃=H; R₂=F; R₅=t-But; R₆=CH₃)

A solution of methyl 2-(3-fluoro-4-morpholinophenylamino)acetate (400 mg, 1.49 mmol), of formula (**VII**), in DMF (14 ml) is added with a catalytic amount of dimethylaminopyridine, and di-t-butylcarbonate (2.928 g, 13.41 mmol). The mixture is heated at 120°C for about 30 minutes. Afterwards, the mixture is diluted with diethyl ether and the organic phase is washed with water. The organic phases are dried over Na₂SO₄ and evaporated under reduced pressure. The product is purified by flash chromatography to obtain 404 mg of product in 67% yield.

¹H NMR (400 MHz, *CDCl*₃) ppm 7.17-6.95 (bs, 2H), 6.89 (t, *J*= 8,5 Hz, 1H), 4.26 (s, 2H), 3.94-3.84 (m, 4H), 3.78 (s, 3H), 3.16-3.02 (m, 4H), 1.46 (s, 9H).

According to a similar procedure, the following compounds are obtained:
- methyl 2-((3-fluoro-4-morpholinophenyl)(ethoxycarbonyl)amino) acetate; and
- methyl 2-((3-fluoro-4-morpholinophenyl)(isobutoxycarbonyl)-amino) acetate.

### Example 4: Synthesis t-butyl 3-fluoro-4-morpholinophenyl-(2-oxoethyl)-carbamate (V; R=R₁=R₃=H; R₂=F; R₅=t-.But)

A solution of 2-((3-fluoro-4-morpholinophenyl)(t-butoxycarbonyl) amino) methyl acetate (404 mg, 1.09 mmol) of formula (**VIII**), in toluene (5.5 ml), cooled at about -78°C and under N₂ atmosphere, is dropwise added with a 1.0 M solution of Dibal-H in CH₂Cl₂ (1.65 ml, 1.65 mmol). Afterwards, the mixture is reacted 1 hour, then 893 µl of methanol are dropped therein, keeping a temperature of about -78°C. The reaction mixture is then left to warm at room temperature and added with a NH₄Cl saturated solution. The aqueous phase is extracted three times with ethyl acetate (the formed Al³⁺ salts should be removed from the resulting mixture by filtration through Celite). The combined organic phases are separated, dried over Na₂SO₄ and evaporated under reduced pressure. The product is recovered by flash chromatography to obtain 315.3 mg of product, in 86% yield.

¹H NMR (400 MHz, *CDCl*₃) ppm 9.70 (s, 1H), 7.14-6.81 (m, 3H), 4.32 (s, 2H), 3.93-3.85 (m, 4H), 3.13-3.05 (m, 4H), 1.46 (s, 9H)

According to a similar procedure, the following compounds are obtained:
- ethyl 3-fluoro-4-morpholinophenyl-(2-oxoethyl)-carbamate; and
- isobutyl 3-fluoro-4-morpholinophenyl-(2-oxoethyl)-carbamate.

### Example 5: Synthesis of N-(2,2-diethoxyethyl)-3-fluoro-4-morpholinoaniline (IX; R=R₁=R₃=H; R₂=F; R₇=C₂H₅)

A solution of methyl 2-(3-fluoro-4-morpholinophenylamino)acetate (200 mg, 1.019 mmol), of formula (**VI**), in CH₃CN (1 ml) is added with diisopropylethylamine (266 µl, 1.528 mmol), chloroacetaldehyde diethylacetal (611 µl, 4.077 mmol) and a catalytic amount of tetrabutylammonium iodide. The solution is microwave heated in a closed vessel, at 170°C for about 30 minutes. After that, acetonitrile is evaporated off under reduced pressure, the residue is taken up with water and extracted 3 times with ethyl acetate. The combined organic phases are dried over Na₂SO₄ and evaporated under reduced pressure. The product is purified by flash chromatography (hexane: acetate 7:3) to a 22% final yield.

¹H NMR (400 MHz, *CDCl*₃) ppm 6.85 (b, 1H), 6.49-6.3 (m, 2H), 4.67 (t, *J*=5.4Hz, 1H), 3.87 (m, 4H), 3.74 (qd, *J*=9.42, 7.08 Hz, 2H), 3.58 (qd, *J*=9.37, 7.02 Hz, 2H), 3.21 (m, 2H), 2.99 (m, 4H), 1.25 (t, *J*= 7.07 Hz, 6H).

### Example 6: Synthesis of N-(2,2-dimethoxyethyl)-3-fluoro-4-morpholinoaniline (IX; R=R₁=R₃=H; R₂=F; R₇=CH₃)

A solution of methyl 2-(3-fluoro-4-morpholinophenylamino)acetate (3 g, 15.289 mmol), of formula (**VI**), in EtOH (61 ml), under N₂ atmosphere, is added with a dimethoxyacetaldehyde aqueous solution 60% weight/weight (3.068 ml, 20.33 mmol) and the mixture is left under stirring for about 2 hours. After that, 300 mg of Pd/C are added and the mixture is kept under H₂ atmosphere at atmospheric pressure. After approximately 12 hours the reaction is completed, the catalyst is filtered through Celite, the residue is washed with ethanol and ethyl acetate, the solvent mixture is evaporated off under reduced pressure, the crude is taken up with ethyl acetate and the organic phase is washed 3 times with H₂O. The combined organic phases are dried over Na₂SO₄ and evaporated under reduced pressure. The resulting product is recrystallized from hexane in a yield higher than 90%.

¹H NMR (400 MHz, *CDCl*₃) ppm 7.04-6.76 (bm, 1H), 6.46-6.34 (m, 2H), 4.56 (t, *J*= 5.5 Hz, 1H), 3.98-3.79 (m, 5H), 3.43 (s, 6H), 3.21 (d, *J* = 5.5 Hz, 2H), 3.07-2.95 (bm, 4H).

### Example 7: Synthesis of ethyl 2,2-diethoxyethyl(3-fluoro-4-morpholino-phenyl) carbamate (X; R=R₁=R₃=H; R₂=F; R₅=C₂H₅; R₇₌CH₂CH₃)

A solution of N-(2,2-diethoxyethyl)-3-fluoro-4-morpholinoaniline (500 mg, 1.758 mmol), of formula (**IX**), in CH₃CN (17.58 ml) is added with diisopropylethylamine (306 µl, 1.758 mmol), ethyl chloroformate (336 µl, 3.517 mmol) and a catalytic amount of dimethylaminopyridine. The mixture is microwave heated in a closed vessel at about 170°C for approximately 30 minutes. After that, a further equivalent of ethyl chloroformate is added and the mixture is heated for a further 30 minutes under the same conditions at about 170°C. Afterwards, acetonitrile is evaporated off under reduced pressure, then the resulting solid is taken up with H₂O (pH should be basic) and the aqueous phase is extracted 3 times with ethyl acetate. The organic phases are dried over Na₂SO₄ and evaporated under reduced pressure. The product is crystallized from MeOH and H₂O in 80% yield.

¹H NMR (400 MHz, *CDCl*₃) ppm 7.16-6.95 (m, 2H), 6.85 (t, *J* = 9.17 Hz, 1H), 4.56 (m, 1H), 4.20-4.01 (m, 2H), 3.80-3.75 (m, 4H), 3.67 (d, 2H), 3.31 (s, 6H), 1.34-1.14 (m, 9H).

According to a similar procedure, the following compounds are obtained:
- isobutyl 2,2-diethoxyethyl(3-fluoro-4-morpholino-phenyl) carbamate; and
- tert-butyl 2,2-diethoxyethyl(3-fluoro-4-morpholino-phenyl) carbamate.

### Example 8: Synthesis of 2,2-dimethoxyethyl(3-fluoro-4-morpholino-phenyl) carbamate of ethyl (X; R=R₁=R₃=H; R₂=F; R₅=C₂H₅; R₇=CH₃)

A solution of N-(2,2-dimethoxyethyl)-3-fluoro-4-morpholinoaniline (25.0 g, 87.9 mmol), of formula (**IX**), in toluene (250 ml) is added with 12.8 g of N,N-diisopropyl ethylamine (99.0 mmol) and heated to 50°C. A solution of 14.3 g of ethyl chloroformate (132 mmol) in 30 ml of toluene is then dropped therein in about 30 minutes. After completion of the addition, the mixture is reacted at 50°C for about 20 minutes, then left to cool, and added with 220 ml of a solution prepared from 9.5 g of 37% HCl and purified water. The phases are separated, the aqueous phase is extracted again with toluene and the combined organic phases are washed with water. The combined organic phases are dried over Na₂SO₄ and evaporated under reduced pressure.

H NMR (400 MHz, *CDCl*₃) ppm 7.16-6.95 (m, 2H), 6.85 (t, *J* = 9.17 Hz, 1H), 4.56 (m, 1H), 4.20-4.01 (m, 2H), 3.90-3.85 (m, 4H), 3.67 (d, 2H), 3.31 (s, 6H), 3.08-3.03 (m, 4H), 1.24-1.14 (m, 3H).

According to a similar procedure, the following compounds are obtained:
- isobutyl 2,2-dimethoxyethyl(3-fluoro-4-morpholino-phenyl) carbamate; and
- tert-butyl 2,2-dimethoxyethyl(3-fluoro-4-morpholino-phenyl) carbamate.

### Example 9: Synthesis of ethyl 3-fluoro-4-morpholinophenyl(2-oxoethyl)carbamate (V; R=R₁=R₃=H; R₂=F; R₅=C₂H₅)

A solution of ethyl 2,2-dimethoxyethyl(3-fluoro-4-morpholinophenyl)carbamate (500 mg, 1.6 mmol), of formula (**X**), in CH₃CN (16 ml) is added with a 3 N HCl aqueous solution (about 500 µl). The solution is then microwave heated in a closed vessel at about 170°C for approximately 30 minutes. After that, the solvent is evaporated off under reduced pressure and the product is purified by flash chromatography (hexane:acetate 6:4) to provide an oil in 60% yield.

¹H NMR (400 MHz, *CDCl*₃) ppm 9.70 (s, 1H), 7.06-6.95 (m, 2H), 6.93-6.86 (m, 1H), 4.38 (s, 2H), 4.20 (q, *J* = 7.1 Hz, 2H), 3.90-3.86 (m, 4H), 3.12-3.07 (m, 4H), 1.25 (t, *J* = 7.0 Hz, 3H).

According to a similar procedure, the following compounds are obtained:
- isobutyl 3-fluoro-4-morpholinophenyl(2-oxoethyl)carbamate; and
- tert-butyl 3-fluoro-4-morpholinophenyl(2-oxoethyl)carbamate.

### Example 10: Synthesis of ethyl 3-fluoro-4-morpholinophenyl(2-oxoethyl)carbamate (V; R=R₁=R₃=H; R₂=F; R₅=C₂H₅)

A solution of ethyl 2,2-dimethoxyethyl-(3-fluoro-4-morpholinophenyl)carbamate (28.5 g, 80 mmol), of formula (**X**), in 9:1 CH₃CN:H₂O (300 ml) is added with 136 g (1.2 mol) of CF₃COOH in about 1.5 h keeping the inner temperature below 30°C. The mixture is reacted for 18 h. After completion of the reaction, the solvent is evaporated off under reduced pressure, repeatedly removing water with toluene. The residue is then taken up with ethyl acetate and water and the aqueous phase is basified with K₂CO3. The phases are separated, the aqueous phase is extracted again with ethyl acetate and the combined organic phases are washed with water. The combined organic phases are dried over Na₂SO₄ and evaporated under reduced pressure. 24.3 g of a light color solid is obtained.

¹H NMR (400 MHz, *CDCl*₃) ppm 9.70 (s, 1H), 7.06-6.95 (m, 2H), 6.93-6.86 (m, 1H), 4.38 (s, 2H), 4.20 (q, *J* = 7.1 Hz, 2H), 3.90-3.86 (m, 4H), 3.12-3.07 (m, 4H), 1.25 (t, *J* = 7.0 Hz, 3H).

According to a similar procedure, the following compounds are obtained:
- isobutyl 3-fluoro-4-morpholinophenyl(2-oxoethyl)carbamate; and
- tert-butyl 3-fluoro-4-morpholinophenyl(2-oxoethyl)carbamate.

### Example 11: Synthesis of racemic ethyl 3-fluoro-4-morpholino-phenyl(2-hydroay-3-nitropropyl) carbamate (III; R=R₁=R₃=H; R₂=F; Rs₌C₂H₅)

66 ul of tetrabutylammonium fluoride, 1 M solution in tetrahydrofuran, equivalent to 0.9 eq, is diluted in tetrahydrofuran (1.5 ml) under nitrogen stream and magnetic stirring. The solution is cooled to about 0°C and the resulting mixture is added with a solution of 2.4 eq of CH₃NO₂ (9.6 µl) in tetrahydrofuran (290 µl). The reaction mixture is left under stirring at about 0°C and a solution of ethyl 3-fluoro-4-morpholinophenyl(2-oxoethyl) carbamate (25 mg, 1 eq), of formula (**V**), in 0.6 ml of tetrahydrofuran is dropped therein under nitrogen stream. After about 20 minutes at 0°C the reaction is completed. The reaction mixture is poured in an ammonium chloride aqueous saturated solution. The aqueous phase is extracted 3 times with ethyl acetate and the combined organic phases are washed with water and subsequently dried over dry Na₂SO₄. The solvent is evaporated off under reduced pressure, then the product is purified by flash chromatography (eluent hexane/acetate 1:1) to obtain an ivory white solid in 75% yield.

H NMR (400 MHz, *CDCl*₃) ppm 7.07-6.87 (m, 3H), 4.59-4.49 (m, 1H), 4.49-4.41 (m, 2H), 4.18 (q, *J* = 7.1 Hz, 2H), 3.92-3.81 (m, 5H), 3.76 (dd, *J* = 14.6, 4.6 Hz, 1H), 3.73-3.53 (b, 1H), 3.15-3.07 (m, 4H), 1.23 (t, *J* = 7.0 Hz, 3H).

According to a similar procedure, the following compounds are obtained:
- racemic isobutyl 3-fluoro-4-morpholinophenyl(2-hydroxy-3-nitropropyl) carbamate; and
- racemic tert-butyl 3-fluoro-4-morpholinophenyl(2-hydroxy-3-nitropropyl) carbamate.

### Example 12: Synthesis of ethyl (S)-3-fluoro-4-morpholinophenyl(2-hydroxy-3-nitropropyl) carbamate (III; R=R₁=R₃=H; R₂=F; R₅=C₂H₅)

A solution of (3aR,3a'R,8aS,8a'S)-2,2'-(propane-2,2-diyl)bis(8,8a-dihydro-3aH-indeno[1,2-d]oxazole) (Indabox; 6 mg, 0.11 eq) and copper acetate hydrate (3 mg, 0.1 eq) in ethanol (240 µl) is prepared, and kept under stirring for 60 minutes; then ethyl 3-fluoro-4-morpholinophenyl(2-oxoethyl)carbamate (50 mg, 1 eq), of formula (**V**), and nitromethane (86.6 µl, 10 eq) are added. After about 6 days, the solvent is evaporated off under reduced pressure and the product is purified by flash chromatography (eluent: CH₂Cl₂/Et₂O 8:2), to obtain an ivory white solid in 66% yield. Chiral HPLC analysis shows that the two enantiomers (S) and (R) are present in a 93.6 to 6.4 ratio.

¹H NMR (400 MHz, *CDCl*₃) ppm 7.07-6.87 (m, 3H), 4.59-4.49 (m, 1H), 4.49-4.41 (m, 2H), 4.18 (q, *J* = 7.1 Hz, 2H), 3.92-3.81 (m, 5H), 3.76 (dd, *J* = 14.6, 4.6 Hz, 1H), 3.73-3.53 (b, 1H), 3.15-3.07 (m, 4H), 1.23 (t, *J* = 7.0 Hz, 3H).

### Example 13: Synthesis of ethyl (S)-3-fluoro-4-morpholinophenyl(2-hydroxy-3-nitropropyl) carbamate (III; R=R₁=R₃=H; R₂=F; R₅=C₂H₅)

A solution of (3aR,3a'R,8aS,8a'S)-2,2'-(propane-2,2-diyl)bis(8,8a-dihydro-3aH-indeno[1,2-d]oxazole) (Indabox; 125 mg, 0.11 eq) and copper acetate hydrate (66 mg, 0.1 eq) in ethanol (5.2 ml) is prepared, and kept under stirring for 60 minutes; then ethyl 3-fluoro-4-morpholinophenyl(2-oxoethyl)carbamate (1.0 g, 3.2 mmol), of formula (**V**), and nitromethane (1.7 ml, 10 eq) are added. After about 7 days, the reaction mixture is concentrated under reduced pressure and the crude diluted in ethyl acetate and washed with a diluted aqueous solution of ammonia. After separation, the organic phase is washed with water, dried over sodium sulfate, filtered and concentrated under reduced pressure to a residue. The crude is purified by crystallization from toluene to give a white solid in 80% yield. Chiral HPLC analysis shows that the two enantiomers (S) and (R) are present in a 94.4 to 5.6 ratio.

¹H NMR (400 MHz, *CDCl*₃) ppm 7.07-6.87 (m, 3H), 4.59-4.49 (m, 1H), 4.49-4.41 (m, 2H), 4.18 (q, *J* = 7.1 Hz, 2H), 3.92-3.81 (m, 5H), 3.76 (dd, *J* = 14.6, 4.6 Hz, 1H), 3.73-3.53 (b, 1H), 3.15-3.07 (m, 4H), 1.23 (t, *J* = 7.0 Hz, 3H).

According to a similar procedure, the following compounds are obtained:
- isobutyl (S)-3-fluoro-4-morpholinophenyl(2-hydroxy-3-nitropropyl) carbamate; and
- tert-butyl (S)-3-fluoro-4-morpholinophenyl(2-hydroxy-3-nitropropyl) carbamate.

The resulting compounds of formula (**III**) have enantiomeric purity of approximately 99.7%.

### Example 14: Synthesis of ethyl (R)-3-fluoro-4-morpholinophenyl(2-hydroxy-3-nitropropyl) carbamate (III; R=R₁=R₃=H; R₂=F; R₅=C₂H₅)

Following a similar procedure to that of Example 12, using a solution of (3aS,3a'S,8aR,8a'R)-2,2'-(propane-2,2-diyl)bis(8,8a-dihydro-3aH-indeno[1,2-d]oxazole), the three compounds of Example 12 are obtained, as (R) enantiomers.

### Example 15: Synthesis of racemic ethyl N-(3-amino-2-hydroxypropyl)-N-(3-fluoro-4-morpholinophenyl) carbamate (IV; R=R₁=R₃=H; R₂=F; R₅₌C₂H₅)

A methanol solution of racemic ethyl 3-fluoro-4-morpholinophenyl(2-hydroxy-3-nitropropyl) carbamate (500 mg, 1 eq), of formula (**III**), is added with 100 mg of 10% Pd/C. The reaction mixture is kept under H₂ atmosphere for about 4 hours. Afterwards, the reaction mixture is filtered through Celite. The celite pad is repeatedly washed with ethyl acetate. The solvent is evaporated off under reduced pressure and the product is purified by flash chromatography (eluent: CH₂Cl₂/MeOH 9:1 1% TEA). The yield is 54%.

¹H NMR (400 MHz, *CDCl*₃) ppm 7.06-6.85 (m, 3H), 4.99-4.50 (b, 3H)(exchange with D₂O), 4.24-4.08 (m, 3H), 3.93-3.85 (m, 4H), 3.82 (dd, *J* = 14.4, 8.0 Hz, 1H), 3.65 (dd, *J* = 14.4, 4.1 Hz, 1H), 3.17-3.04 (m, 5H), 3.04-2.90 (m, 1H), 1.21 (t, *J* = 6.9 Hz, 3H).

According to a similar procedure, the following compounds are obtained:
- racemic isobutyl N-(3-amino-2-hydroxypropyl)-N-(3-fluoro-4-morpholinophenyl) carbamate; and
- racemic tert-butyl N-(3-amino-2-hydroxypropyl)-N-(3-fluoro-4-morpholinophenyl) carbamate.

According to a similar procedure, starting from the (R) or (S) enantiomers of the respective compounds of formula (**III**), the (S) or (R) enantiomers of the compounds of formula (**IV**) are respectively obtained.

### Example 16: Synthesis of racemic ethyl N-(3-acetamido-2-hydroxypropyl)-N-(3-fluoro-4-morpholinophenyl) carbamate (II; R=R₁=R₃=H; R₂=F; R₄=CH₃; R₅=C₂H₅)

A solution of racemic ethyl 3-amino-2-hydroxypropyl(3-fluoro-4-morpholinophenyl) carbamate (50 mg, 1 eq), of formula (**IV**), in 1.5 ml of CH₂Cl₂, under nitrogen atmosphere, is added with 13.2 µl, 1 eq of acetic anhydride and 19.48 µl, 1 eq of triethylamine. After 2 hours the reaction mixture is poured in water and the aqueous phase is extracted 3 times with CH₂Cl₂; the combined organic phases are dried over dry Na₂SO₄. The solvent is evaporated off under reduced pressure and the product is recovered by flash chromatography (eluent: acetate). The yield is 25%.

¹H NMR (400 MHz, *CDCl*₃) ppm 7.07-6.84 (m, 3H), 4.25-4.09 (m, 2H), 4.08-3.96 (m, 1H), 3.94-3.84 (m, 4H), 3.79 (dd, *J* = 14.6, 7.7 Hz, 1H), 3.69 (dd, *J* = 14.6, 4.0 Hz, 1H), 3.18-3.04 (m, 5H), 2.96 (dd, *J* = 13.3, 8.1 Hz, 1H), 2.09 (s, 3H), 1.23 (t, *J* = 7.0 Hz, 3H).

According to a similar procedure, the following compounds are obtained:
- isobutyl N-(3-acetamido-2-hydroxypropyl)-N-(3-fluoro-4-morpholinophenyl) carbamate; and
- tert-butyl N-(3-acetamido-2-hydroxypropyl)-N-(3-fluoro-4-morpholinophenyl) carbamate.

According to a similar procedure, starting from the (R) or (S) enantiomers of the respective compounds of formula (**IV**), the (R) or (S) enantiomers of the compounds of formula (**II**) are obtained respectively.

### Example 17: Synthesis of racemic N-[[3-(3-fluoro-4-morpholinyl)-phenyl]-2-oxo-5-oxazolidinyl]methyl]acetamide, (I; R=R₁=R₃=H; R₂=F; R₄₌CH₃)

A solution of 10 mg of racemic ethyl 3-acetamido-2-hydroxypropyl(3-fluoro-4-morpholinophenyl) carbamate, of formula (**II**), in tetrahydrofuran, under nitrogen stream at room temperature, is dropped into a round-bottom flask containing 1 mg of NaH. After about 90 minutes the reaction is completed. An ammonium chloride aqueous saturated solution is added and the aqueous phase is extracted 3 times with ethyl acetate. The combined organic phases are dried over dry Na₂SO₄ and the solvent is evaporated off under reduced pressure. The product is recovered by flash chromatography (eluent acetate/MeOH 9:1) in 30% yield.

¹H NMR (400 MHz, *CDCl*₃) ppm 7.47 (dd, *J* = 14.4, 2.6 Hz, 1H), 7.13-7.07 (m, 1H), 6.98 (t, *J* = 9.1 Hz, 1H), 6.13 (t, *J* = 5.9 Hz, 1H), 4.83-4.75 (m, 1H), 4.04 (t, *J* = 9.0 Hz, 1H), 3.93-3.87 (m, 4H), 3.80-3.68 (m, 2H), 3.68-3.58 (m, 1H), 3.12-3.05 (m, 4H), 2.04 (s, 3H).

### Example 18: Synthesis of racemic N-[[3-(3-fluoro-4-morpholinyl)-phenyl]-2-oxo-5-oxazolidinyl]methyl]acetamide, (I; R=R₁=R₃=H; R₂=F; R₄₌CH₃)

A solution of 4.8 g (12.5 mmol) of racemic ethyl 3-acetamido-2-hydroxypropyl(3-fluoro-4-morpholinophenyl) carbamate, of formula (**II**), in ethanol (35 ml) is added with potassium t-butoxide (1.5 g, 12.7 mmol) and the mixture is reacted at room temperature. After about 20 minutes, a solid precipitates which is filtered and washed with ethanol, to obtain 2.1 g of product (50% yield).

¹H NMR (400 MHz, *CDCl*₃) ppm 7.47 (dd, *J* = 14.4, 2.6 Hz, 1H), 7.13-7.07 (m, 1H), 6.98 (t, *J* = 9.1 Hz, 1H), 6.13 (t, *J* = 5.9 Hz, 1H), 4.83-4.75 (m, 1H), 4.04 (t, *J* = 9.0 Hz, 1H), 3.93-3.87 (m, 4H), 3.80-3.68 (m, 2H), 3.68-3.58 (m, 1H), 3.12-3.05 (m, 4H), 2.04 (s, 3H).

According to a similar procedure, the following compounds are obtained:
starting from the (R) or (S) enantiomer of an alkyl carbamate of formula (II), obtained in example 15, the title compound is obtained as the single (R) or (S) enantiomer.

The resulting compounds of formula (**I**), have enantiomeric purity approximately equal to 99.7% and mean particle size D₅₀ of about 50 micrometres.

## Claims

1. A process for the preparation of a compound of formula (**I**), or a salt thereof, as the single (S) or (R) enantiomer, or as a mixture thereof, 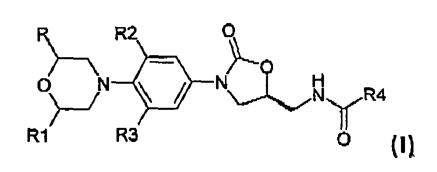 wherein R and R₁, which can be the same or different, are hydrogen, CH₃, CN, COOH or COOC₁-C₄ alkyl;
R₂ and R₃, which can be the same or different, are hydrogen, F or Cl; and
R₄ is C₁-C₄ alkyl;
which process comprises:
- the reduction of a compound of formula (**III**), or a salt thereof, as the single (S) or (R) enantiomer or as a mixture thereof, 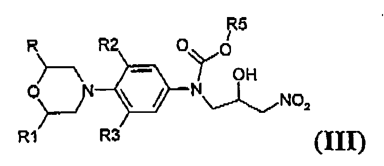 wherein R₅ is optionally substituted C₁-C₄ alkyl, aryl-C₁-C₄ alkyl or aryl; and R-R₄ are as defined above; to obtain a compound of formula (**IV**), or a salt thereof, as the single (S) or (R) enantiomer or as a mixture thereof, 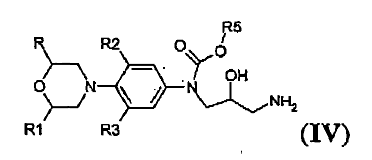 wherein R to R₃ and R₅ are as defined above;
- the acylation thereof to obtain a compound of formula (**II**), as the single (S) or (R) enantiomers, or as a mixture thereof, or a salt thereof, 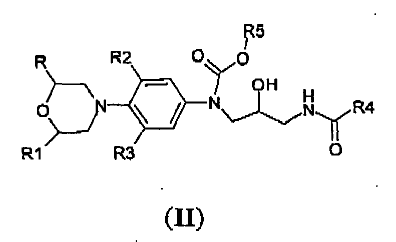 wherein R to R₅ are as defined above and, if the case, its conversion to a salt thereof, or *vice versa;* and
a) the activation of the hydroxyl group in a compound of formula (**II**), as the single (S) or (R) enantiomer, or as a mixture thereof, or a salt thereof, by means of a derivatization reagent to form a leaving group, and
subsequent cyclization reaction in the presence of a catalyst or an azide compound; or
b) the cyclization reaction of a compound of formula (**II**), or a salt thereof, as the single (S) or (R) enantiomer, or as a mixture thereof, in presence of a strong base; and, if desired, the resolution of a mixture of enantiomers of formula (**I**) to the single (S) or (R) enantiomer; and/or, if desired, the conversion of a compound of formula (**I**) to a salt thereof, or *vice versa* **characterized in that**
c) a compound of formula (**III**), or a salt thereof, as a mixture of the two enantiomers, is obtained starting from an achiral compound of formula (V), or a salt thereof, 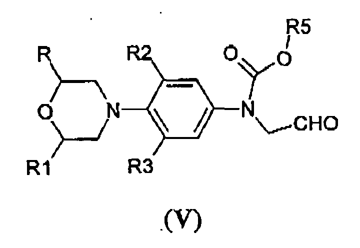 wherein R₁ to R₃ and R₅ are as defined above;
by reaction with nitromethane in the presence of a base and, optionally, in the presence of a solvent; or
d) a compound of formula (**III**), or a salt thereof, as a single (S) or (R) enantiomer is obtained starting from an achiral compound of formula (**V**), or a salt thereof,
by reaction with nitromethane in the presence of an optically pure catalyst and, optionally, in the presence of a solvent.

2. A process as claimed in claim 1, wherein in the compound of formula (**I**) R, R₁ and R₃ are hydrogen; R₂ is fluorine and R₄ is methyl.

3. A process as claimed in claim 1, step a), wherein the catalyst is a Lewis acid; and an azide compound is sodium azide.

4. A process as claimed in claim 3, wherein the catalyst is selected from BF₃, AlCl₃, ZnCl₂, trifluoromethanesulfonic acid, trifluoroacetic acid, acetic acid, trimethylsilyl triflate, tert-butyldimethylsilyl triflate.

5. A process as claimed in claim 1, step a), wherein the derivatizing agent is a sulfonyl chloride of formula R₅SO₂Cl, wherein R₅ is as defined in claim 1; an alkyl or aryl phosphine and an azadicarboxylate; or carbonyl diimidazole.

6. A process as claimed in claim 1, step b), wherein the strong base is selected from an alkali C₁-C₆ alkoxide, a tertiary amine, a carbanion, an azanion, sodium hydride, sodium hexamethyldisilazide and potassium hexamethyldisilazide.

7. A process as claimed in claim 1 step c), wherein the base is an organic base, preferably tetrabutylammonium fluoride or diazabicycloundecene.

8. A process as claimed in claim 1, for the preparation of a compound of formula (**I**), or: a salt thereof, as the single (S) or (R) enantiomer, as defined in claim 1, wherein a compound of formula (**III**), or a salt thereof, as the single (S) or (R) enantiomer is obtained starting from an achiral compound of formula (**V**), or a salt thereof, by reaction with nitromethane in the presence of an optically pure catalyst and, optionally, in the presence of a solvent.

9. A process as claimed in claim 1 step d), or in claim 8, wherein an optically pure chiral catalyst is a complex of Cu(II) with a bisoxazolidinc, preferably (3aR,3a'R,8aS,8a'S)-2,2'-(propane-2,2-diyl)bis8(8,8a-dihydro-3aH-indeno[1,2-d]oxazole) or (1R,2S)-Inda-Box.

10. A compound of formula (**II**), or a salt thereof, as a single (S) or (R) enantiomer, or as a mixture thereof 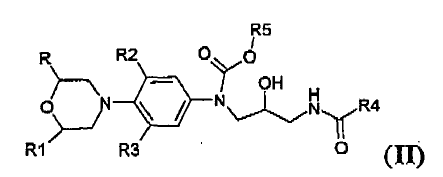 wherein R and R₁, which can be the same or different,are hydrogen, CH₃, CN, COOH or COOC₁-C₄ alkyl; R₂ and R₃, which can be the same or different, are hydrogen, F or Cl; R₄ is C₁-C₄ alkyl and R₅ is benzyl, t-butyl, isopropyl, isobutyl, methyl or ethyl.

11. A compound of formula (**II**), as claimed in claim 10, which is selected from:
• ethyl N-(3-acetamido-2-hydroxy-propyl)-N-(3-fluoro-4-morpholinophcnyl) carbamate;
• t-butyl N-(3-acetamido-2-hydroxy-propyl)-N-(3-fluoro-4-morpholinophenyl) carbamate; and
• isobutyl N-(3-acetamido-2-hydroxy-propyl)-N-(3-fluoro-4-morpholinophenyl) carbamate.

12. A process as claimed in claim 1, which comprises utilizing, as starting material a compound of formula (V), or a salt thereof, 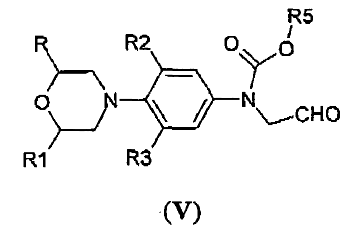 wherein R to R₃ and R₅ are as defined in claim 1.

13. A process according to claim 12, wherein a compound of formula (**V**) is selected from:
• ethyl 3-fluoro-4-morpholinophenyl-(2-oxoethyl)-carbamate;
• isobutyl 3-fluoro-4-morpholinophenyl-(2-oxoethyl)-carbamate; and
• t-butyl 3-fluoro-4-morpholiophenyl-2-oxoethyl)-carbamate.

14. A process as claimed in claim 1, which comprises utilizing, as starting material a compound of formula (**III**), or of formula (**IV**), or a salt thereof, as the single (R) or (S) enantiomer, or as a mixture thereof, 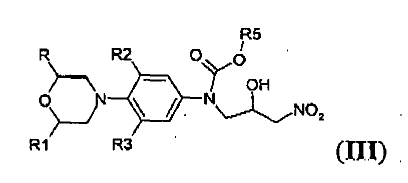 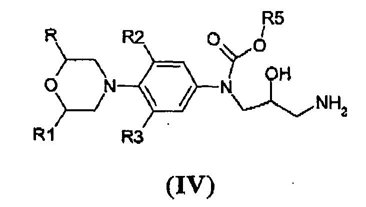 wherein R to R₃ and R₅ are as defined in claim 1.

15. A process as claimed in claim 14, wherein a compound of formula (**III**) is selected from:
• ethyl N-(3-fluoro-4-morpholinophenyl)-N-(2-hydroxy-3-nitiopropyl) carbamate;
• isobutyl N-(3-fluoro-4-morpholinophenyl)-N-(2-hydroxy-3-nitropropyl) carbamate; and
• t-butyl N-(3-fluoro-4-morpholinophenyl)-N-(2-hydroxy-3-nitropropyl) carbamate; as the single (R) or (S) enantiomer, or as mixtures thereof; and
a compound of formula (IV) is selected from:
• ethylN-(3-amino-2-hydroxypropyl)-N-(3-fluoro-4-morpholinophenyl)-carbamate;
• isobutyl N-(3-amino-2-hydroxypropyl)-N-(3-fluoro-4-morpholinophenyl)-carbamate; and
• t-butylN-(3-amino-2-hydroxypropyl)-N-(3-fluoro-4-morpholinophenyl)-carbamate; as the single (R) or (S) enantiomer, or as mixtures thereof.

## Patentansprüche

1. Verfahren zur Herstellung einer Verbindung der Formel (I) oder eines Salzes davon als einzelnes (S)- oder (R)-Enantiomer oder als Gemisch davon, 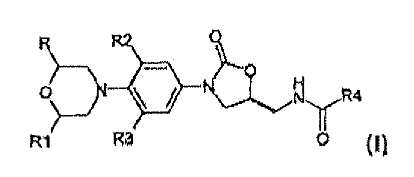 worin R und R₁, die gleich oder verschieden sein können, Wasserstoff, CH₃, CN, COOH oder COOC₁-C₄-Alkyl sind;
R₂ und R₃, die gleich oder verschieden sein können, Wasserstoff, F oder Cl sind, und
R₄ C₁-C₄-Alkyl ist;
wobei das Verfahren umfasst:
- Reduktion einer Verbindung der Formel (III) oder eines Salzes davon als einzelnes (S)- oder (R)-Enantiomer oder als Gemisch davon, 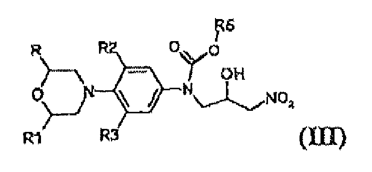 worin R₅ gegebenenfalls substituiertes C₁-C₄-Alkyl, Aryl-C₃-C₄-alkyl oder Aryl ist und R-R₄ wie oben definiert sind; unter Erhalt einer Verbindung der Formel (IV) oder eines Salzes davon als einzelnes (S)- oder (R)-Enantiomer oder als Gemisch davon 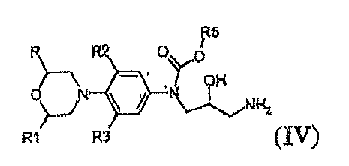 worin R bis R₃ und R₅ wie oben definiert sind;
- Acylierung davon unter Erhalt einer Verbindung der Formel (**II**) als einzelnes (S)- oder (R)-Enantiomer oder als Gemisch davon oder eines Salzes davon, 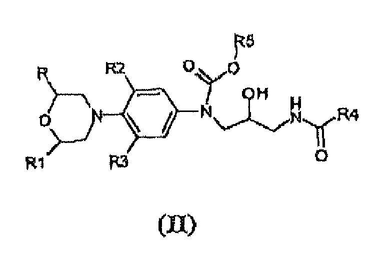 worin R bis R₅ wie oben definiert sind, und gegebenenfalls ihre Umwandlung in ein Salz davon oder vice versa; und
a) Aktivierung der Hydroxylgruppe in einer Verbindung der Formel (**II**) als einzelnes (S)- oder (R)-Enantiomer oder als Gemisch davon oder einem Salz davon mit Hilfe eines Derivatisierungsreagenzes unter Bildung einer Abgangsgruppe und
anschließende Cyclisierungsreaktion in Gegenwart eines Katalysators oder einer Azidverbindung oder
b) Cyclisierungsreaktion einer Verbindung der Formel (**II**) oder eines Salzes davon als einzelnes (S)- oder (R)-Enantiomer oder als Gemisch davon in Gegenwart einer starken Base und, wenn gewünscht, Auftrennung eines Gemisches von Enantiomeren der Formel (I) in das einzelne (S)- oder (R)-Enantiomer und/oder, wenn gewünscht, Umwandlung einer Verbindung der Formel (I) in ein Salz davon oder vice versa, **dadurch gekennzeichnet, dass**
c) eine Verbindung der Formel (III) oder ein Salz davon ausgehend von einer achiralen Verbindung der Formel (V) oder einnem Salz davon 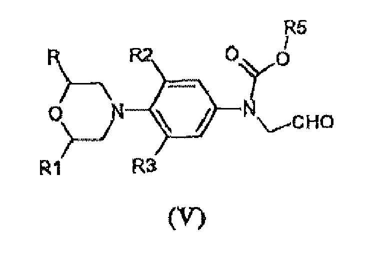 worin R₁ bis R₃ und R₅ wie oben definiert sind;
durch Reaktion mit Nitromethan in Gegenwart einer Base und gegebenenfalls in Gegenwart eines Lösungsmittels erhalten wird oder
d) eine Verbindung der Formel (III) oder ein Salz davon als einzelnes (S)- oder (R)-Enantiomer aus einer achiralen Verbindung der Formel (V) oder einem Salz davon durch Reaktion mit Nitromethan in Gegenwart eines optisch reinen Katalysators und gegebenenfalls in Gegenwart eines Lösungsmittels erhalten wird.

2. Verfahren, wie es in Anspruch 1 beansprucht wird, wobei in der Verbindung der Formel (**I**) R, R₁ und R₃ Wasserstoff sind; R₂ Fluor ist und R₄ Methyl ist.

3. Verfahren, wie es in Anspruch 1, Schritt a) beansprucht wird, wobei der Katalysator eine Lewis-Säure ist und eine Azidverbindung Natriumazid ist.

4. Verfahren, wie es in Anspruch 3 beansprucht wird, wobei der Katalysator aus BF₃, AlCl₃, ZnCl₂, Trifluormethansulfonsäure, Trifluoressigsäure, Essigsäure, Trimethylsilyltriflat, tert-Butyldimethylsilyltriflat ausgewählt wird.

5. Verfahren, wie es in Anspruch 1, Schritt a) beansprucht wird, wobei das Derivatisierungsmittel ein Sulfonylchlorid der Formel R₅SO₂Cl, worin R₅ wie in Anspruch 1 definiert ist, ein Alkyl- oder Arylphosphin und ein Azadicarboxylat oder Carbonyldiimidazol ist.

6. Verfahren, wie es in Anspruch 1, Schritt b) beansprucht wird, wobei die starke Base aus einem Alkali-C₁-C₆-alkoxid, einem tertiären Amin, einem Carbanion, einem Azanion, Natriumhydrid, Natriumhexamethyldisilazid und Kaliumhexamethyldisilazid ausgewählt wird.

7. Verfahren, wie es in Anspruch 1, Schritt c) beansprucht wird, wobei die Base eine organische Base, vorzugsweise Tetrabutylammoniumchlorid oder Diazabicycloundecen, ist.

8. Verfahren, wie es in Anspruch 1 beansprucht wird, zur Herstellung einer Verbindung der Formel (I) oder eines Salzes davon als einzelnes (S)- oder (R)-Enantiomer, wie in Anspruch 1 definiert, wobei eine Verbindung der Formel (III) oder ein Salz davon als einzelnes (S)- oder (R)-Enantiomer ausgehend von einer achiralen Verbindung der Formel (V) oder einem Salz davon durch Reaktion mit Nitromethan in Gegenwart eines optisch reinen Katalysators und gegebenenfalls in Gegenwart eines Lösungsmittels erhalten wird.

9. Verfahren, wie es in Anspruch 1, Schritt d) oder in Anspruch 8 beansprucht wird, wobei ein optisch reiner chiraler Katalysator ein Komplex von Cu(II) mit einem Bisoxazolidin, vorzugsweise (3aR,3a'R,8aS,8a'S)-2,2'-(Propan-2,2-diyl)bis-8-(8,8a-dihydro-3aH-indeno[1,2-d]oxazol) oder (1R,2S)-Inda-Box, ist.

10. Verbindung der Formel (**II**) oder ein Salz davon als einzelnes (S)- oder (R)-Enantiomer oder als Gemisch davon 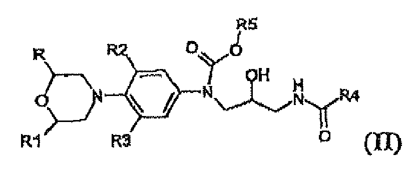 worin R und R₁, die gleich oder verschieden sein können, Wasserstoff, CH₃, CN, COOH oder COOC₁-C₄-Alkyl sind; R₂ und R₃, die gleich oder verschieden sein können, Wasserstoff, F oder Cl sind; R₄ C₁-C₄-Alkyl ist und R₅ Benzyl, t-Butyl, Isopropyl, Isobutyl, Methyl oder Ethyl ist.

11. Verbindung der Formel (**II**), wie sie in Anspruch 10 beansprucht wird, die ausgewählt wird aus:
• Ethyl-N-(3-acetamido-2-hydroxy-propyl)-N-(3-fluor-4-morpholinophenyl)carbamat;
• t-Butyl-N-(3-acetamido-2-hydroxy-propyl)-N-(3-fluor-4-morpholinophenyl)carbamat und
• Isobutyl-N-(3-acetamido-2-hydroxy-propyl)-N-(3-fluor-4-morpholinophenyl)carbamat.

12. Verfahren, wie es in Anspruch 1 beansprucht wird, das Verwenden einer Verbindung der Formel (V) oder eines Salzes davon 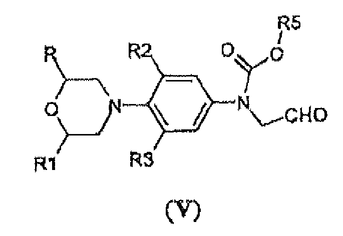 worin R bis R₃ und R₅ wie in Anspruch 1 definiert sind, als Ausgangsmaterial umfasst.

13. Verfahren gemäß Anspruch 12, wobei eine Verbindung der Formel (V) ausgewählt wird aus:
• Ethyl-3-fluor-4-morpholinophenyl-(2-oxoethyl)-carbamat;
• Isobutyl-3-fluor-4-morpholinophenyl-(2-oxoethyl)-carbamat und
• t-Butyl-3-fluor-4-morpholinophenyl-(2-oxoethyl)-carbamat.

14. Verfahren, wie es in Anspruch 1 beansprucht wird, das Verwenden einer Verbindung der Formel (III) oder der Formel (IV) oder eines Salzes davon als einzelnes (R)- oder (S)-Enantiomer oder als Gemisch davon als Ausgangsmaterial umfasst: 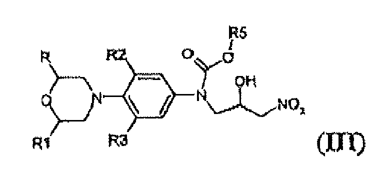 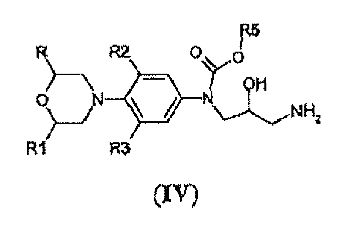 worin R bis R₃ und R₅ wie in Anspruch 1 definiert sind.

15. Verfahren, wie es in Anspruch 14 beansprucht wird, wobei eine Verbindung der Formel (III) ausgewählt wird aus
• Ethyl-N-(3-fluor-4-morpholinophenyl)-N-(2-hydroxy-3-nitropropyl)carbamat;
• Isobutyl-N-(3-fluor-4-morpholinophenyl)-N-(2-hydroxy-3-nitropropyl)carbamat und
• t-Butyl-N-(3-fluor-4-morpholinophenyl)-N-(2-hydroxy-3-nitropropyl)carbamat
als einzelnes (R)- oder (S)-Enantiomer oder als Gemische davon, und
eine Verbindung der Formel (IV) ausgewählt wird aus:
• Ethyl-N-(3-amino-2-hydroxypropyl)-N-(3-fluor-4-morpholinophenyl)-carbamat;
• Isobutyl-N-(3-amino-2-hydroxypropyl)-N-(3-fluor-4-morpholinophenyl)-carbamat und
• t-Butyl-N-(3-amino-2-hydroxypropyl)-N-(3-fluor-4-morpholinophenyl)-carbamat
als einzelnes (R)- oder (S)-Enantiomer oder als Gemische davon.

## Revendications

1. Procédé de préparation d'un composé de formule (I), ou d'un sel de celui-ci, sous forme de l'énantiomère (S) ou (R) seul, ou d'un mélange des deux, 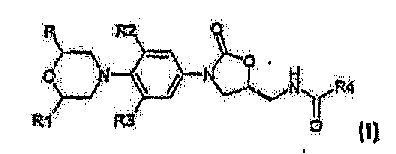 dans laquelle R et R₁, qui peuvent être identiques ou différents, sont un atome d'hydrogène, CH₃, CN, COOH ou COO-alkyle en C₁-C₄ ;
R₂ et R₃, qui peuvent être identiques ou différents, sont un atome d'hydrogène, F ou Cl ; et
R₄ est un alkyle C₁-C₄
lequel procédé comprend :
- la réduction d'un composé de formule (III), ou d'un sel de celui-ci, sous forme de l'énantiomère (S) ou (R) seul, ou d'un mélange des deux, 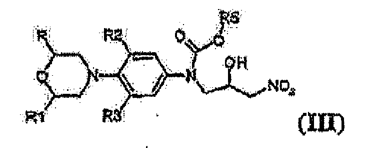 dans laquelle R₅ est un alkyle en C₁-C₄, un aryl-alkyle en C₁-C₄ ou un aryle éventuellement substitué ; et R-R₄ sont tels que définis ci-dessus ; pour obtenir un composé de formule (**IV**), ou un sel de celui-ci, sous forme de l'énantiomère (S) ou (R) seul, ou d'un mélange des deux, 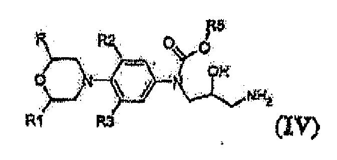 dans laquelle R à R₃ et R₅ sont tels que définis ci-dessus ;
- l'acylation de ce dernier pour obtenir un composé de formule (**II**), sous forme de l'énantiomère (S) ou (R) seul, ou d'un mélange des deux, ou d'un sel de celui-ci, 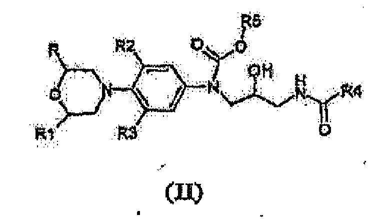 dans laquelle R à R₅ sont tels que définis ci-dessus et, le cas échéant, sa conversion en un sel de celui-ci, ou *inversement;* et
a) l'activation du groupe hydroxyle dans une composé de formule (**II**), sous forme de l'énantiomère (S) ou (R) seul, ou d'un mélange des deux, ou d'un sel de celui-ci, à l'aide d'un réactif de formation de dérivés pour former un groupe partant, et
une réaction de cyclisation ultérieure en présence d'un catalyseur ou d'un composé azoture ; ou
b) la réaction de cyclisation d'un composé de formule (**II**), ou d'un sel de celui-ci, sous forme de l'énantiomère (S) ou (R) seul, ou d'un mélange des deux, en présence d'une base forte ; et, si souhaité, le dédoublement d'un mélange des énantiomères de formule (I) en énantiomère (S) ou (R) seul; et/ou, si souhaité, la conversion d'un composé de formule (I) en un sel de celui-ci, ou *inversement,* **caractérisé en ce que**
c) un composé de formule (**III**), ou un sel de ceiui-ci, sous forme d'un mélange des deux énantiomères, est obtenu en partant d'un composé achiral de formule (V), ou d'un sel de celui-ci, 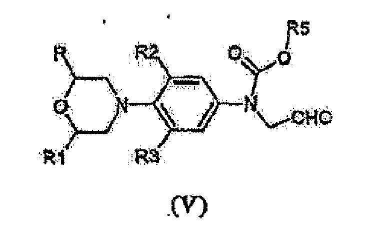 dans laquelle R₁ à R₃ et R₅ sont tels que définis ci-dessus ;
par réaction avec le nitromethane en présence d'une base et, en option, en présence d'un solvant ; ou
d) un composé de formule (III), ou un sel de celui-ci, sous forme de l'énantiomère (S) ou (R) seul est obtenu en partant d'un composé achiral de formule (V), ou d'un sel de celui-ci,
par réaction avec le nitromethane en présence d'un catalyseur optiquement pur et, en option, en présence d'un solvant.

2. Procédé tel que revendiqué dans la revendication 1 dans lequel, dans le composé de formule (**I**), R, R₁ et R₃ sont un atome d'hydrogëne ; R₂ est un atome de fluor et R₄ est un méthyle.

3. Procédé tel que revendiqué dans la revendication 1, étape a), dans lequel le catalyseur est un acide de Lewis ; et le composé azoture est l'azoture de sodium.

4. Procédé tel que revendiqué dans la revendication 3, dans lequel le catalyseur est choisi parmi BF₃, AlCl₃, ZnCl₂, l'acide trifluorométhanesulfonique, l'acide trifluoroacétique, l'acide acétique, le triflate de triméthylsilyle, et le triflate de tert-butyldiméthylsilyle.

5. Procédé tel que revendiqué dans la revendication 1, étape a), dans lequel l'agent de formation de dérivés est un chlorure de sulfonyle de formule R₅SO₂Cl, où R₅ est tel que défini dans la revendication 1 ; une alkyl,- ou une arylphosphine et un azadicarboxylate ; ou un carbonyldiimidazole.

6. Procédé tel que revendiqué dans la revendication 1, étape b), dans lequel la base forte est choisie parmi un alcoxyde en C₁-C₆ alcalin, une amine tertiaire, un carbanion, un azanion, un hydrure de sodium, un hexaméthyldisilazide de sodium et un hexaméthyldisilazide de potassium.

7. Procédé tel que revendiqué dans la revendication 1, étape c), dans lequel la base est une base organique, de préférence, un fluorure de tétrabutylammonium ou un diazabicyclaundécène.

8. Procédé tel que revendiqué dans la revendication 1, pour la préparation d'un composé de formule (I), ou d'un sel de celui-ci, sous forme de l'énantiomère (S) ou (R) seul, tel que défini dans la revendication 1, dans lequel un composé de formule (**III**), ou un sel de celui-ci, sous forme de l'énantiomère (S) ou (R) seul est obtenu en partant d'un composé achiral de formule (V), ou d'un sel de celui-ci, par réaction avec le nitromethane en présence d'un catalyseur optiquement pur et, en option, en présence d'un solvant.

9. Procédé tel que revendiqué dans la revendication 1, étape d), ou dans la revendication 8, dans lequel le catalyseur chiral optiquement pur est un complexe de Cu(II) avec une bisoxazolidine, de préférence, un (3aR,3a'R,8aS,8a'S)-2,2'-(propane-2,2-diyl)bis8(8,8a-dihydro-3aH-indéno[1,2-d]oxazole) ou (1R,2S)-Inda-Box.

10. Composé de formule (II), ou un sel de celui-ci, sous forme d'un énantiomère (S) ou (R) seul, ou d'un mélange des deux 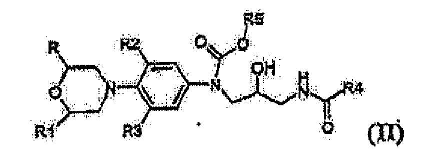 dans laquelle R et R₁, qui peuvent être identiques ou différents, sont un atome d'hydrogène, CH₃, CN, COOH ou COO-alkyle en C₁-C₄ ; R₂ et R₃, qui peuvent être identiques ou différents, sont un atome d'hydrogène, F ou Cl ; R₄ est un alkyle en C₁-C₄ et R₅ est un benzyle, un t-butyle, un isopropyle, un isobutyle, un méthyle ou un éthyle.

11. Composé de formule (**II**) selon la revendication 10, qui est choisi parmi :
• le N-(3-acétamido-2-hydroxy-propyl)-N-(3-fluoro-4-morpholinophényl)carbamate d'éthyle ;
• le N-(3-acétamido-2-hydroxy-propyl)-N-(3-fluoro-4-morpholinophényl)carbamate de t-butyle ; et
• le N-(3-acétamido-2-hydroxy-propyl)-N-(3-fluoro-4-morpholinophényl)carbamate d'isobutyle,

12. Procédé selon la revendication 1, qui comprend l'utilisation, à titre de réactif de départ, d'un composé de formule (V), ou d'un sel de celui-ci, 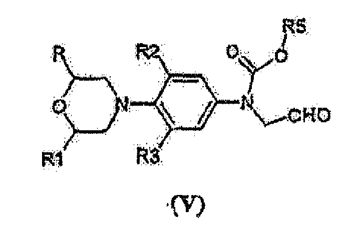 dans laquelle R à R₃ et R₅ sont tels que définis dans la revendication 1.

13. Procédé selon la revendication 12, dans lequel un composé de formule (V) est choisi parmi :
• le 3-fluoro-4-morpholinophényl-(2-oxoéthyl)-carbamate d'éthyle ;
• le 3-fluoro-4-morpholinophényl-(2-oxoéthyl)-carbamate d'isobutyle ; et
• le 3-fluoro-4-morpholinophényl-(2-oxoéthyl)-carbamate de t-butyle.

14. Procédé tel que revendiqué dans la revendication 1, qui comprend l'utilisation, à titre de réactif de départ, d'un composé de formule (III), ou de formule (**IV**), ou d'un sel de celui-ci, sous forme de l'énantiomère (R) ou (S) seul, ou d'un mélange des deux, 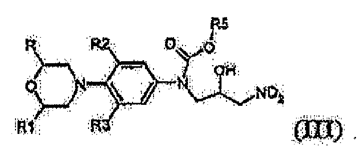 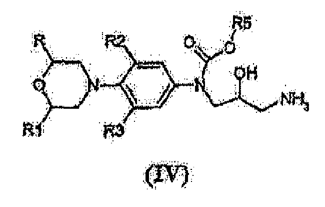 dans laquelle R à R₃ et R₅ sont tels que définis dans la revendication 1.

15. Procédé tel que revendiqué dans la revendication 14, dans lequel un composé de formule (III) est choisi parmi:
• le N-(3-fluoro-4-inorpholinophényl)-N-(2-hydroxy-3-nitropropyl)carbamate d'éthyle ;
• le N-(3-Fluoro-4-morpholinophényl)-N-(2-hydroxy-3-nitropropyl)carbamate d'isobutyle ; et
• le N-(3-fluoro-4-morpholinophényl)-N-(2-hydroxy-3-nitropropyl)carbamate de t-butylc ; sous forme de l'énantiomère (R) ou (S) seul, ou de mélanges des deux ; et
un composé de formule (**IV**) est choisi parmi:
• le N-(3-amino-2-hydroxypropyl)-N-(3-fluoro-4-morpholinophényl)carbamate d'éthyle ;
• le N-(3-amino-2-hydroxypropyl)-N-(3-fluoro-4-morpholinophényl)carbamate d'isobutyle ; et
• le N-(3-amino-2-hydroxypropyl)-N-(3-fluoro-4-morpholinophényl)carbamate de t-butyle ; sous forme de l'énantiomère (R) ou (S) seul, ou de mélanges des deux.
